Europäisches Patentamt

European Patent Office

Office européen des brevets

(11) Publication number: **0 165 973 B1**

## (12) EUROPEAN PATENT SPECIFICATION

(45) Date of publication of patent specification: **11.03.92**

(51) Int. Cl.⁵: **A61K  37/26**, B67D 5/48

(21) Application number: **85900396.4**

(22) Date of filing: **10.12.84**

(86) International application number:
**PCT/US84/02020**

(87) International publication number:
**WO 85/02544 (20.06.85 85/14)**

(54) INSULIN DELIVERY ALGORITHM.

(30) Priority: **16.12.83 US 562435**
         **10.12.84 US 678511**

(43) Date of publication of application:
**02.01.86 Bulletin  86/01**

(45) Publication of the grant of the patent:
**11.03.92 Bulletin  92/11**

(84) Designated Contracting States:
**AT DE FR GB NL SE**

(56) References cited:

**No relevant documents have been disclosed.**

(73) Proprietor: **AOKI, Thomas Takemi**
**1021 El Sur Way**
**Sacramento, CA 95825(US)**

(72) Inventor: **AOKI, Thomas Takemi**
**1021 El Sur Way**
**Sacramento, CA 95825(US)**

(74) Representative: **Patentanwälte Grünecker,**
**Kinkeldey, Stockmair & Partner**
**Maximilianstrasse 58**
**W-8000 München 22(DE)**

# Description

This invention relates to a programmable insulin infusion pump for controllably timing the administration of insulin and various fuels (carbohydrates, proteins, and fats) to individuals whose fuel processing (i.e. oxidation, storage, and conversion to other substrates) capabilities are chronically abnormal (e.g. diabetic subjects) or acutely disrupted (e.g. accidentally or surgically traumatized patients).

The normal fuel processing capabilities of the body, such as of the liver and other tissues, e.g., muscle, can be disrupted by disease (e.g., diabetes) and by trauma (accidental or surgical).

Insulin is often administered in large quantities to diabetic subjects, surgical patients or accident (especially burn) victims in attempts to restore normal fuel processing capabilities and to prevent the breakdown of muscle protein. Since massive doses of insulin lower blood sugar levels, glucose may also be infused.

Established treatment programs for diabetic patients administer insulin to control blood glucose concentrations. Insulin infusion patterns based directly on measured blood glucose levels are disclosed in Aisenberg et al., U.S. Patent No. 3,387,339; Klein, U.S. Patent No. 4,206,755; Edelman, U.S. Patent No. 4,073,292; and Chem. Abstracts, 92:135345m. Insulin infusion patterns based on projected blood glucose levels and expected changes in blood glucose levels, calculated using measured blood glucose levels, are disclosed in Albisser et al., U.S. Patent No. 4,245,634; Schindler et al, U.S. Patent No. 4,253,456; Clemens, U.S. Patent No. 4,151,845; Clemens et al, U.S. Patent No. 4,055,175; and Chem. Abstracts, 89:39926u. In programs of the above types, both the amount of insulin infused and the duration of the infusion are dependent on measured glucose concentrations. That is, when measured glucose concentrations and/or projected concentrations arise to a certain prescribed limit, insulin is administered. Insulin infusion then continues until such glucose concentrations approach or fall below a certain level.

In addition to these patterns of insulin infusion based on concurrently-measured glucose levels, insulin-administering devices have been developed which can infuse insulin according to a predetermined profile of the patient's insulin requirements. For example, Franetzki et al, U.S. Patent No. 4,482,872, discloses such a device wherein a base rate of insulin is continuously infused and the predetermined program (stored in a microprocessor), which typically administers a larger amount (pulse) of insulin, can be called up by the patient when needed. Haerten et al, U.S. Patent No. 4,077,405, likewise discloses a device which can administer pulses of medication (e.g., insulin) over a constant baseline in response to pre-programmed or manually-controlled signals. These devices administer insulin in patterns which, like the above-mentioned methods which respond to concurrently-measured glucose concentrations, are a function of and are designed to directly control the actual concentration of glucose in the blood stream.

## Summary of the Invention

According to the present invention the programmable insulin infusion pump used in the treatment of a diabetic or a traumatized subject comprises :
a programmable microprocessor;
a pump mechanism controlled by said microprocessor;
an insulin reservoir feeding into said pump mechanism; and
a catheter extending from said pump mechanism capable of terminating in a subject intravascularly intraperitoneally, or subcutaneously,
said microprocessor being programmed to cause said pump to administer insulin pulses to a diabetic or traumatized subject during at least part of a period in which an elevated carbohydrate concentration is established in said subject's metabolic system, said pulses arranged to produce a series of peaks in the free insulin concentration of the metabolic system of said subject, spaced such that the minima between said peaks are successively increasing, thereby activating the fuel processing capabilities of said subject's body tissues.

In preferred embodiments the changes in the free insulin concentration are effected by administering time-varying quantities of insulin, the elevated carbohydrate concentration is established by the ingestion or infusion of carbohydrates, the administering step is initiated coincident with the establishing step, the insulin is administered in pulses, a primary series of insulin pulses which produces sharp peaks over a gradually increasing interpeak concentration is followed by an interval during which no insulin is administered to allow the insulin concentration to return to that concentration which existed prior to the administration of the primary series of pulses (the baseline concentration), the interval is followed by the administration of a secondary series of insulin pulses to produce an insulin concentration which oscillates about the baseline concentration which maintains the fuel processing system in an active state, the average amount of insulin in the pulses of the secondary series is less than the average amount of insulin in the pulses of the primary series, and the secondary series continues during the entire period between carbohydrate ingestions or infusions.

The program of changes in insulin concentrations of the present invention activates and maintains processing capabilities for body fuels such as carbohydrates (glucose, fructose, sucrose, galactose, starches, and related analogs and derivatives), amino acids, and lipids of a diabetic or a traumatized patient's system. In diabetic patients, the method of insulin administration indirectly controls blood glucose concentrations by addressing the underlying problem of diabetes, namely the absence of autoregulation of glucose concentrations by the metabolic system, by rapidly and efficiently activating and maintaining the metabolically-dormant dietary carbohydrate processing system, in particular the liver but including muscle.

Other features and advantages of the invention will be apparent from the following description of the preferred embodiment thereof, and from the claims.

Description of the Preferred Embodiment

We first briefly describing the drawings.

Drawings

Fig. 1 shows the concentrations of "free" insulin achieved by the preferred embodiments of the invention.

Fig. 2 is an enlargement of Section A of Fig. 1.

Fig. 3 is an enlargement of Section C of Fig. 1.

Fig. 4 is a schematic block diagram of an external programmable insulin pump programmed to deliver insulin according to the present invention.

Fig. 5 is a schematic block diagram of an implantable programmable insulin pump programmed to deliver insulin according to the present invention.

Description

The present program of insulin concentration changes is directed to coordinating elevated carbohydrate concentrations in body tissues, for example due to the ingestion of a carbohydrate-containing meal or due to a carbohydrate (e.g., glucose) infusion, with the insulin concentrations resulting from the program so that they may act together to activate and maintain the body's fuel processing capabilities. In particular, the program is designed to coordinate elevated liver and portal vein glucose concentrations with the aforementioned insulin concentrations so as to activate the dietary carbohydrate processing system which, based on investigations into the enzymes involved in the metabolism of glucose and the distribution of exogenous and endogenous glucose in the metabolic system, is believed to be primarily in the liver and to a lesser extent in muscle. Both the amount of insulin infused and the duration of the administration of the insulin pulses are independent of prevailing blood glucose levels.

Due to the binding of insulin to antibodies in persons who has received insulin (e.g., insulin-taking diabetic subjects), the concentration of insulin which is available to interact so as to regulate fuel processing capabilities, in particular to regulate blood glucose concentrations, is typically much lower than the total concentration of insulin in the system. The concentration of insulin which is so available is referred to as the "free" insulin concentration.

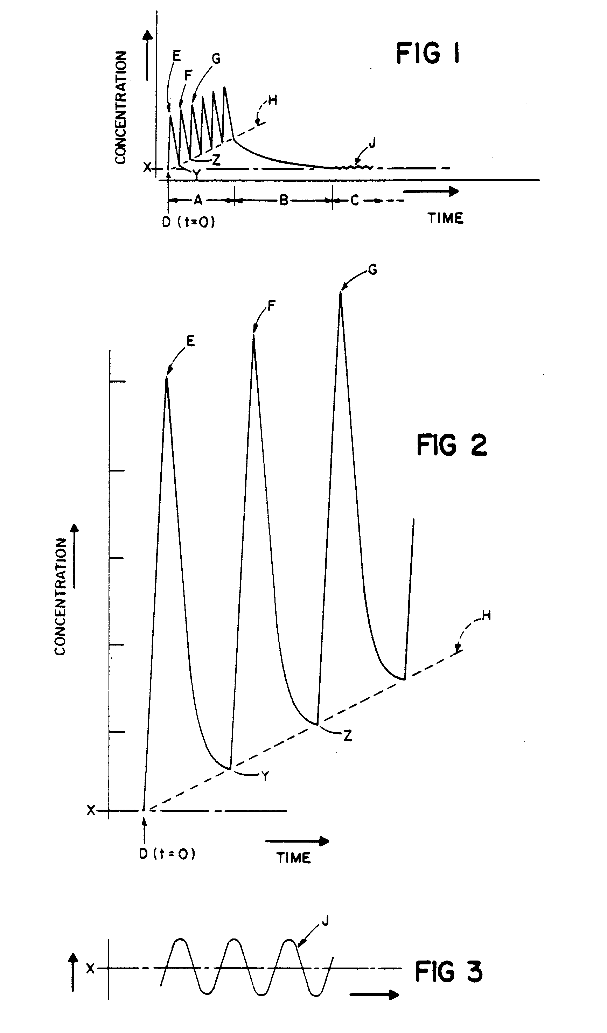

The initial portion of the program is designed to activate the body's fuel processing capabilities, in particular the dietary carbohydrate processing system. In order to activate the system, the body's tissues, in particular the liver (but including muscle), must see (i.e., be subject to) sharp changes in the "free" insulin concentration as well as a gradual overall increase in the interpeak "free" insulin concentration, at the same time that the concentration of glucose in the liver and portal vein is high, e.g., after ingestion of a carbohydrate-containing meal or after an infusion of carbohydrates. Section A of Fig. 1 and Fig. 2 show the necessary pattern of "free" insulin concentrations. The combination of these three factors, i.e., rapid changes in "free" insulin concentration superimposed over a gradually increasing "free" insulin concentration in the presence of a high intra-liver or portal vein glucose concentration, will cause the liver (and to a lesser extent muscle) to synthesize and activate the enzymes responsible for metabolizing glucose.

After the system is activated, it may be maintained by administering time-varying quantities of insulin to produce a "free" insulin concentration which oscillates near or about the baseline concentration, i.e., that concentration of insulin which existed prior to administering the initial portion of the program. Section C of Fig. 1 and Fig. 3 show the oscillating "free" insulin concentrations.

The time-varying quantities of insulin are preferably pulses of insulin, i.e., injections or infusions which start and stop within a short period of time (on the order of seconds). However, any time-varying quantities which produce the necessary pattern of "free" insulin concentrations may be used.

The administration of a primary series of pulses of insulin spaced closely enough such that the effect of one pulse (i.e., a "free" insulin concentration peak) has not been completely dissipated before the next pulse is administered will result in the necessary sharp changes in "free" insulin concentration over an increasing interpeak insulin concentration. Referring to Figs. 1 and 2, a carbohydrate-containing meal is ingested or a carbohydrate infusion is initiated at D, or time = 0, when the "free" insulin concentration is X. X represents the concentration of "free" insulin present in the patient's system prior to the administration of the insulin pulses of the primary series, and is herein referred to as the baseline concentration. For example, in a diabetic patient X would be the lowest "free" insulin concentration in the patient's system following the last insulin injection of a typical treatment program. Typical baseline "free" insulin concentrations are from 5 to 15 micro-Units of insulin per milliliter of serum ($\mu$U/ml).

Coincident with or shortly following the establishment of an elevated carbohydrate concentration, the first pulse of the primary series of the present insulin delivery program is administered, which will result in peak E. The pulse is an amount of insulin sufficient to cause the peak "free" insulin concentration in the blood to reach from 50 to 3000 $\mu$U/ml, preferably 100 to 2000 $\mu$U/ml. When the "free" insulin concentration decreases by about 90% to Y (i.e., to about 10% over the "free" insulin concentration at the time of administering the first pulse) the second pulse of the primary series is administered, which will result in peak F. When the "free" insulin concentration again decreases by about 90% to Z, or to about 10% over the "free" insulin concentration at the time of administering the second pulse, the next pulse of the primary series is administered, which will result in peak G. Repetition of this process will result in the increasing interpeak "free" insulin concentration denoted by line H. In the primary series of insulin pulses the amount of insulin injected per pulse may be constant or may vary provided the peak "free" insulin concentratin achieved after each pulse is from 50 to 3000 $\mu$U/ml, preferably 100 to 2000 $\mu$U/ml. The interpulse duration also may be constant or may vary, provided the next subsequent pulse is administered before the insulin concentration resulting from the previous pulse has returned to that concentration which existed prior to administering the previous pulse, so that the interpeak "free" insulin concentration increases by 10 to 500 $\mu$U/ml from one pulse to the next. The duration of the primary series administered with each meal or carbohydrate infusion does not exceed three hours and generally falls within the range of about 6 to 180 minutes. Particularly effective results have been obtained with a series of 10 pulses, administered six minutes apart, over an interval of 56 minutes. Since it is desirable to administer the least amount of insulin consistent with activation of dietary fuel processing system, and since the amount of insulin required to activate a system will vary from patient to patient or even from day to day in the same patient, a rigid length or duration cannot be assigned to the primary series.

After completion of the pre-determined primary insulin pulses--carbohydrate meal/infusion sequence, pulse administration is suspended to allow the "free" insulin concentration to return to the baseline concentration, as shown in Section B of Fig. 1.

When the free insulin concentration is at or near the baseline concentration, a secondary series of smaller insulin pulses is administered to produce a concentration of "free" insulin which oscillates about or near the baseline concentration, as shown by curve J in Section C of Fig. 1 and in Fig. 3. The pulses of insulin of the secondary series are of an amount sufficient to result in peak "free" insulin concentrations of 10-300 $\mu$U/ml. The pulses (single or paired) are spaced so as to maintain a relatively constant interpeak "free" insulin concentration of 5 to 15 $\mu$U/ml between discrete pulses or pulse pairs. That is, the effects of one pulse (or pair of pulses) are allowed to dissipate before the next pulse (or pulse pair) is administered.

Administration of this secondary series will (1) together with the primary series, maintain in an active state the body's fuel processing capabilities, in particular the dietary carbohydrate processing system, and (2) permit cycling of hepatic glucose output and hepatic glucose uptake in the period between carbohydrate ingestions or infusions. That is, as the "free" insulin concentration increases above the baseline the glucose-producing function of the activated liver is inhibited and the liver will take up glucose; as the "free" insulin concentration falls towards or below the baseline the activated liver will produce and release glucose.

Once the fuel processing system has been activated and maintained for a period of time (from one to four days) by the primary and secondary series of the insulin infusion program, it is enabled to function in a normal manner. In diabetic patients, repetitive administration of the primary and secondary series over this period enables the liver (and muscle) to function normally to autoregulate body glucose concentrations. While in insulin-dependent diabetic patients a permanent cessation of insulin injections or infusions is not possible, it is anticipated that once the program has been administered for about one to four days the activated dietary fuel processing system would only require a "tune-up" every seven to 30 or more days. Between "tune-ups" the diabetic subject would be returned to a standard conventional therapy (e.g., subcutaneous insulin, oral hypoglycemic agents, diet) in order to maintain basic levels of insulin in the metabolic system. However, with an activated fuel processing system coupled to standard conventional therapy, the diabetic subject would be less subject to wide fluctuations in blood sugar levels and would need a less restrictive American Diabetes Association diet. Alternatively, the system may be maintained indefinitely by administering the primary series with meals and the secondary series during the nighttime.

An insulin infusion program which conforms to the present invention would involve, for example, administering a primary series of insulin pulses (e.g., intravascularly (including the portal vein), intraperitoneally, or subcutaneously) immediately following the ingestion of a mixed meal containing 10-100 g of dietary carbohydrate of alteratively 10-100 g of glucose or its equivalent (e.g., Sustacal), or the infusion of an equivalent synthetic combination of fuels. The pulses of the primary series, administered every six to thirty minutes over a 6 to 180 minute period, may be of equal or variable amounts with the average amount of the pulses being between 0.01-0.05 Units of insulin per kilogram of body weight (U/kg), and preferably 0.02 to 0.04 U/kg. As previously stated, it is desired to use the least amount of insulin required to obtain the desired therapeutic effect. These pulses will produce a corresponding series of peaks in the "free" insulin concentration having a peak amplitude of 50-3000 $\mu$U/ml, preferably 100-2000 $\mu$U/ml, in arterial blood or in arterialized venous blood at six to thirty minute intervals. Coincident with these "free" insulin peaks the interpeak "free" insulin concentration will increase by 10-500 $\mu$U/ml at six to thirty minute intervals, achieving a maximum interpeak "free" insulin concentration of 60-1000 $\mu$U/ml at 6 to 180 minutes. Insulin administration is then suspended for an amount of time (e.g., 90-120 minutes) sufficient to allow the "free" insulin concentration to gradually return to the baseline "free" insulin concentration of 5-15 $\mu$U/ml. Following achievement of baseline "free" insulin concentrations, equal or variable insulin pulses of an average amount between 0.001-0.02 U/kg are administered every two to ninety minutes in order to achieve peak "free" insulin concentrations in arterial or arterialized venous blood of 10-300 $\mu$U/ml with a periodicity of two to ninety minutes. This secondary pulse format is continued during the entire period between carbohydrate ingestions or infusions.

The described sequence is repeated with each meal or infusion. The meals are ingested or the infusions are initiated frequently, e.g., from two to eight times a day. Meals and infusions may be administered in any combination, e.g., exclusively meals, exclusively infusions, or meals alternating with infusions in a regular or irregular pattern.

In 8 to 96 hours a significant improvement in the respiratory quotient and carbohydrate oxidation rate should be observed following the ingestion of a carbohydrate-containing meal or an equivalent carbohydrate infusion, observations which are comparable to those seen in normal subjects following ingestion of an equivalent carbohydrate-containing meal or initiation of a comparable infusion.

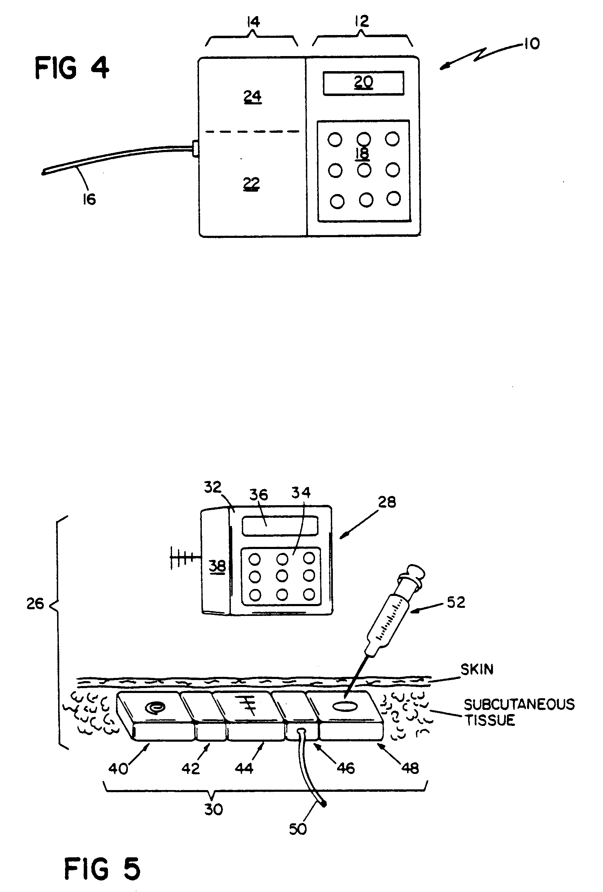

The primary and secondary series of the described insulin delivery program can be infused using a standard external programmable insulin pump, such as shown schematically in Fig. 4. An external pump configuration would be preferable for use in clinical situations, for example, on an out-patient treatment basis (e.g., for "tune-ups"), for accidentally or surgically traumatized patients, or to bring a patient's metabolic system under control prior to surgery. Referring now to Fig. 4, external programmable pump 10 has a programmable microprocessor 12, pump section 14, and infusion catheter 16. Programmable microprocessor 12 includes programming keyboard 18 and display 20, for example an LCD or LED display. Pump section 14 includes pump mechanism 22 and insulin reservoir 24. Infusion catheter 16 extends from pump mechanism 22 and is inserted into the patient to deliver insulin intravascularly (including the portal vein), intraperitoneally, or subcutaneously. For example, in this configuration programmable microprocessor 12 would be programmed (1) to deliver the insulin pulses of the primary series every six to thirty minutes for 6 to 180 minutes, (2) to suspend insulin infusion for 90-120 minutes, and (3) to deliver the smaller insulin pulses of the secondary series until the next primary series is initiated. Initiation of the primary series can be automatically controlled, e.g., programmed to coincide with pre-set carbohydrate infusions, or can be manually controlled, e.g., by pressing the appropriate key on keyboard 18 when a meal is ingested. Alternatively, microprocessor 12 can be programmed to deliver only the primary series (under either manual or automatic control).

Glucose levels can be independently monitored using fingersticks, venipuncture, or glucose sensors. A glucose sensor or analyzer could also be incorporated into the external programmable pump and arranged so as to sound an alarm when glucose levels reach certain pre-set limits as is known in the art, for example, in artificial $\beta$-cells. In this configuration, however, the glucose sensor would not control the initiation or rate of insulin infusion, but would at most be used to suspend insulin infusion if glucose levels reach a pre-set lower limit. Simultaneously with the suspension of insulin infusion, an alarm would alert the patient (or attendant); the insulin infusion program (primary series) could then be manually restarted, if desired, along with the administering of carbohydrates.

The described program can also be administered using a standard implantable programmable insulin pump, such as shown schematically in Fig. 5. The implantable pump configuration would be preferable for long-term self-care by diabetic patients. Referring to Fig. 5, implantable pump assembly 26 includes an external transmitter/receiver unit 28 and the implantable pump unit 30. External transmitter/receiver unit 28 includes programmable microprocessor 32, having programming keyboard 34 and display 36, for example, an LCD or LED display, and telemetry unit 38 which transmits control signals received from microprocessor 32 to implantable unit 30 and transmits informational signals received from implantable unit 30 to microprocessor 32. Implantable pump unit 30 includes power supply 40, programmable microprocessor 42, receiver/transmitter 44 which transmits informational signals received from microprocessor 42 to external unit 28 and transmits control signals received from external unit 28 to microprocessor 42, pump mechanism 46, and insulin reservoir 48. Infusion catheter 50 extends from pump mechanism 46 and terminates intravascularly (including the portal vein), intraperitoneally, or subcutaneously. Insulin reservoir 48 can be refilled using syringe 52. Implantable pump unit 30 can be initially programmed to administer the primary and secondary insulin series as described for the external insulin pump configuration. Using external transmitter/receiver unit 28, the insulin delivery pattern can then be initiated and/or changed, for instance, to deliver only the primary series whenever desired.

Glucose levels can be independently monitored as with the external pump configuration using fingersticks, venipuncture, or glucose sensors. Glucose sensors may also be incorporated into the implantable insulin pump and arranged so as to transmit glucose levels to the external unit 28, as is well known in the art. The external unit could merely sound an alarm when glucose levels reach a pre-settable limit, or could be programmed to suspend insulin infusion (as with the external pump configuration) and sound an alarm simultaneously. It is important to note that in both the external and implantable configurations, the glucose-level-triggered alarm is used primarily for informational purposes and would not independently initiate insulin or glucose infusions.

It should also be noted that administering carbohydrates so as to induce elevational levels sufficient to work in combination with the described insulin-infusion pattern runs directly contrary to standard treatment regimens for diabetic patients. Further, although the pulses are timed to coincide with the anticipated elevated portal vein glucose concentrations following the ingestion of carbohydrate-containing meals or following the initiation of carbohydrate infusions, the pattern of insulin infusion and the resulting insulin concentrations of the present invention differ significantly from those which occur in normal man and from those which occur in diabetic patients on a typical treatment program.

The program of insulin infusion described can be used in diabetic subjects for rapid and efficient primary activation of the fuel processing system, in particular, the dietary carbohydrate processing system, or, for maintenance of the system by the administration, immediately prior to or following the ingestion of meals, of defined intravenous pulses of insulin of smaller magnitude than that used for primary activation. This insulin delivery pattern could also be effective in preserving and/or restoring fuel (e.g., glucose, amino acids, lipids) processing capabilities of hepatic and other tissues (e.g., muscle) in both diabetic and non-diabetic subjects in acute care situations, i.e., traumatized individuals (surgical patients, accident victims) or patients on hyperalimentation.

Other embodiments are within the following claims. For example, the interprandial (including night-time) "free" insulin concentration may be maintained at or near the baseline level by substituting standard insulin therapy for the secondary series, for example, using long-acting (NPH) insulin or using continuous low-level insulin infusion, administering the primary series of pulses with meals; and the primary and/or secondary series could be administered intravenously (including the portal vein), intraperitoneally, or subcutaneously by using multiple oscillatory insulin injection regimens.

## Claims

1. A programmable insulin infusion pump comprising :

   a programmable microprocessor ;

   a pump mechanism controlled by said microprocessor ;

   an insulin reservoir feeding into said pump mechanism ; and

   a catheter extending from said pump mechanism capable of terminating in a subject intravascularly, intraperitoneally, or subcutaneously ;

   said microprocessor being programmed to cause said pump to administer insulin pulses to a diabetic or traumatized subject during at least part of a period in which an elevated carbohydrate concentration is established in said subject's metabolic system, said pulses arranged to produce a series of peaks in the free insulin concentration of the metabolic system of said subject, spaced such that the minima between said peaks are successively increasing, thereby activating the fuel processing capabilities of said subject's body tissues.

2. The pump of Claim 1 wherein said microprocessor, said pump mechanism, and said insulin reservoir are external to said subject.

3. The pump of Claim 1 wherein said microprocessor, said pump mechanism, said catheter, and said insulin reservoir comprise an implantable unit, said implantable unit being implanted subcutaneously in said subject, said microprocessor comprising a first microprocessor, said pump further comprising an external transmitter/receiver unit, said external unit comprising a second programmable microprocessor, means for receiving control signals from said second microprocessor and transmitting said control signals to said implantable unit, and means for receiving informational signals from said implantable unit and transmitting said informational signals to said second microprocessor, said implantable unit further comprising a power supply, said power supply powering said first microprocessor and said pump, means for receiving said control signals from said external unit and transmitting said control signals to said first microprocessor, and means for receiving said informational signals from said first microprocessor and transmitting said informational signals to said external unit.

4. The pump of any of Claims 1, 2, or 3 further comprising a glucose sensor and an audible or visual alarm, said sensor arranged so as to measure said subject's blood glucose level and to activate said alarm and suspend said program of insulin administering when said measured glucose level reaches a pre-set level.

**Revendications**

1. Pompe programmable pour l'administration d'insuline comprenant :
un microprocesseur programmable ;
un mécanisme de pompe commandé par ledit microprocesseur ;
un réservoir d'insuline s'alimentant dans ledit mécanisme de pompe ; et
un cathéter partant du mécanisme de pompe avec terminaison pour introduction intravasculaire, intrapéritonéale ou sous-cutanée dans un sujet ;

ledit microprocesseur étant programmé pour commander à ladite pompe d'administrer des impulsions d'insuline à un sujet diabétique ou traumatisé pendant au moins une partie d'une période dans laquelle une concentration élevée d'hydrates de carbone est établie dans le système métabolique du sujet, lesdites impulsions étant organisées pour produire une série de pics dans la concentration d'insuline libre du système métabolique du sujet, espacés de telle manière que les minimums entre les pics augmentent successivement, ce qui active les capacités de traitement des aliments énergétiques par les tissus dudit sujet.

2. Pompe selon la revendication 1, dans laquelle ledit microprocesseur, ledit mécanisme de pompe, et ledit réservoir d'insuline sont externes au sujet.

3. Pompe selon la revendication 1, dans laquelle ledit microprocesseur, ledit mécanisme de pompe, ledit cathéter et ledit réservoir d'insuline comprennent une unité implantable, cette unité implantable étant implantée en sous-cutané chez le sujet, ledit microprocesseur comprenant un premier microprocesseur, ladite pompe comprenant en outre une unité émettrice/réceptrice externe, cette unité externe comprenant un second microprocesseur programmable, des moyens destinés à recevoir des signaux de commande dudit second microprocesseur et à transmettre ces signaux de commande à ladite unité implantable et des moyens destinés à recevoir des signaux d'information de ladite unité implantable et à transmettre ces signaux d'information audit second microprocesseur, ladite unité implantable comprenant en outre une alimentation en courant, cette alimentation en courant alimentant ledit premier microprocesseur et ladite pompe, des moyens destinés à recevoir lesdits signaux de commande de ladite unité externe et à transmettre ces signaux de commande audit premier microprocesseur et des moyens destinés à recevoir lesdits signaux d'information dudit premier microprocesseur et à transmettre ces signaux d'information à ladite unité externe.

4. Pompe selon l'une quelconque des revendications 1, 2, ou 3, comprenant en outre un capteur de glucose et une alarme sonore ou visuelle, ce capteur étant disposé de manière à mesurer le taux de glucose sanguin du sujet et à déclencher l'alarme et à interrompre le programme d'administration d'insuline lorsque le taux de glucose mesuré atteint un niveau préétabli.

**Patentansprüche**

1. Eine programmierbare Insulininfusionspumpe mit:

   einem programmierbaren Mikroprozessor;

   einer durch den Mikroprozessor gesteuerten Pumpanlage;

   einem die Pumpanlage versorgenden Insulinvorrat; und

   einem sich von der Pumpanlage erstreckenden Katheter, welches in einem Patienten intravaskulär, intraperitonal oder subkutan endet;

   wobei der Mikroprozessor programmiert ist, um die Pumpanlage zum Verabreichen von Insulinpulsen an diabetische oder traumatisierte Patienten während wenigstens eines Teils einer Periode, in der eine erhöhte Kohlehydratkonzentration im Metabolismus des Patienten etabliert ist, zu veranlassen; die Pulse so angeordnet sind, daß eine Reihe von Maxima in der freien Insulinkonzentration des Metabolismus des Patienten auftritt, die so voneinander beabstandet sind, daß die zwischen dem Maxima auftretenden Minima sukzessive anwachsen, wodurch die Brennstoff-Verarbeitungsmöglichkeiten des Körpergewebes des Patienten aktiviert werden.

2. Die Pumpe nach Anspruch 1, **dadurch gekennzeichnet,** daß der Mikroprozessor, die Pumpanlage und der Insulinvorrat extern zum Patienten angeordnet sind.

3. Die Pumpe nach Anspruch 1, **dadurch gekennzeichnet,** daß der Mikroprozessor, die Pumpanlage, das Katheter und der Insulinvorrat eine implantierbare Einheit umfassen, die subkutan in dem Patienten implantierbar ist; der Mikroprozessor einen ersten Mikroprozessor umfaßt, die Pumpe außerdem eine externe Sender-Empfänger-Einheit umfaßt, diese externe Einheit einen zweiten programmierbaren Mikroprozessor, eine Einrichtung zum Empfangen von Steuersignalen von dem zweiten Mikroprozessor und zum Übertragen der Steuersignale zu der implantierten Einheit und eine Einrichtung zum Empfangen von Informationssignalen von der implantierbaren Einheit und zum Übertragen der Informationssignale zu dem zweiten Mikroprozessor umfaßt; die implantierbare Einheit weiterhin eine Kraftquelle, die den ersten Mikroprozessor und die Pumpe antreibt, eine Einrichtung zum Empfangen des Steuerungssignals von der externen Einheit und zum Übertragen des Steuerungssignals zum ersten Mikroprozessor und eine Einrichtung zum Empfangen der Informationssignale von dem ersten Mikroprozessor und zur Übertragung der Informationssignale zur externen Einheit umfaßt.

4. Die Pumpe nach einem der Ansprüche 1, 2 oder 3, **dadurch gekennzeichnet,** daß die Pumpe weiterhin einen Glucosesensor und einen hörbaren oder sichtbaren Alarm umfaßt; der Sensor zur Messung des Blutglucosepegels des Patienten und zur Aktivierung des Alarms angeordnet ist, wobei der Sensor das Programm zur Insulinverabreichung unterbricht, falls der gemessene Glucosepegel einen vorbestimmten Pegel erreicht.

FIG 1

FIG 2

FIG 3

FIG 4

FIG 5